# EUROPÄISCHE PATENTANMELDUNG

(11) **EP 2 859 877 A1**
(43) Veröffentlichungstag der Anmeldung: **15.04.2015**
(21) Anmeldenummer: 14169846.4
(22) Anmeldetag: 26.05.2014
(51) Int. Cl.: A61K 8/03, A61K 8/31, A61K 8/365, A61Q 19/00

(54) **Naturkosmetik-konforme Mehr-Phasen-Zubereitungen enthaltend Guaiazulen**

(30) Priorität: 31.05.2013 DE 102013210188
(71) Anmelder: Beiersdorf AG, 20253 Hamburg (DE)
(72) Erfinder: Triller, Benjamin, DE - 22941 Jersbek (DE); Hüttl, Matthias, DE - 50968 Köln (DE)

(57) **Zusammenfassung**

Die Erfindung ist eine naturkosmetik-konforme Mehr-Phasen-Zubereitungen enthaltend Guaiazulen.

## Beschreibung

Die Erfindung ist eine naturkosmetik-konforme Mehr-Phasen-Zubereitungen enthaltend Guaiazulen.

Kosmetische Mehr- bzw. Zwei-Phasen-Zubereitungen werden vor Gebrauch durch Schütteln vermischt. Nach dem Gebrauch entmischen sich die Produkte innerhalb kurzer Zeit. Das Vorliegen der getrennten Phasen wird dem Verbraucher etwa durch unterschiedliche Färbung der Phasen angezeigt.

Kosmetische Zweiphasenprodukte sind beispielsweise im Bereich der Haarpflege und - styling oder als Make-up Entferner bekannt. Sie bestehen aus einem bestimmten Anteil einer Öl- und einer Wasserphase.

Im Bereich der Reinigungsprodukte hat sich gezeigt, dass zweiphasige Systeme eine bessere Reinigungsleistung haben, besonders bei wasserfestem Make-up. Hier wird die gut reinigende Wirkung von Ölen mit Wasser kombiniert, welches die ölige Sensorik minimiert.

Um ein zwei Phasenprodukt zu bilden, bedarf es einer Phase mit Wasser und einer Phase mit Öl. Im Ruhezustand sind diese beiden Phasen idealerweise optisch klar voneinander getrennt zu erkennen. Durch die unterschiedlichen Dichten vermischen sich diese beiden Phasen nicht selbstständig miteinander und bilden dadurch ein horizontal getrenntes 2-phasiges System aus. Durch das Einbringen von Energie z.B. durch Schütteln, kann man die Phasen kurzzeitig miteinander vermischen. Aufgrund des Dichteunterschiedes und einer hohen Grenzflächenspannung (GFS) von Öl gegenüber Wasser kommt es allerdings danach wieder zu einer sehr schnellen Auftrennung in die zwei Phasen.

Bei der Zugabe von oberflächenaktiven Substanzen, wie Emulgatoren oder Tensiden, muss darauf geachtet werden, dass diese die Zubereitung nicht ausreichend stabilisieren. Durch die Zugabe von Emulgatoren/Tensiden in entsprechenden Konzentrationen könnte ansonsten eine Emulsion erzeugt werden. Der Zusatz an Emulgatoren oder Tensiden kann auch dazu führen, dass sich Teile des Produktes durch das Schütteln dauerhaft emulgieren und so die Öl- und Wasserphase teilweise oder sogar komplett weiß emulgiert bleibt.

Da jedoch die 2-Phasigkeit beabsichtigt ist, muss auf die gezielte Auswahl und Menge der ggf. zugesetzten oberflächenaktiven Substanzen geachtet werden.

Um ein Zwei-Phasen Produkt vermarkten zu können, ist es wichtig, dass eine Phase eingefärbt vorliegt, damit der Konsument sieht, dass er das Produkt vor der Anwendung schütteln muss. Dazu wird meist Ölphase eingefärbt, da sie oberhalb der Wasserphasse ist und es so optisch attraktiver ist.

Zur Einfärbung werden bekanntermassen häufig synthetische Farbstoffe eingesetzt.

Besonders blaue Farbstoffe gelten als sehr selten.

Neben der Einfärbeproblematik ist es das Ziel, die entwickelte Rezeptur möglichst hautfreundlich zu gestalten. Dies gelingt vorteilhaft durch den Einsatz naturkonformer Rohstoffe.

In der Naturkosmetik werden grundsätzlich drei verschiedene Rohstoffklassen unterschieden. Zum einen sind dies natürliche Rohstoffe, die nur durch physikalische Mittel (z.B. Kaltpressen) gewonnen werden. Die zweite Klasse sind naturnahe Rohstoffe. Diese beinhalten chemisch veränderte natürliche Rohstoffe, wobei die chemischen Reaktionen ein Vorbild in der Natur haben (z.B. Verseifung, Veresterung). Zur Sicherstellung der mikrobiellen Sicherheit des Produktes können auch naturidentische d.h. vollsynthetische Konservierungsstoffe eingesetzt werden, die eine dritte Klasse an Naturkosmetikrohstoffen darstellen. Deren Gewinnung aus der Natur ist zu aufwendig. Naturidentische Rohstoffe sind synthetisch hergestellt jedoch mit dem natürlich vorkommenden Rohstoff identisch. Hierunter fallen insbesondere organische Säuren.

Beispiele bekannter natürlicher Rohstoffe sind die in der Tabelle 1 angeführten Stoffe.

**Tabelle 1: Naturkosmetikrohstoffe**

| **Konservierungsstoff** | **Beispiele für Vorkommen in der Natur** |
|---|---|
| Ameisensäure und ihr Natriumsalz | Vorkommen in Insekten seit 1670 bekannt; wird von Käfern und anderen Gliedertieren als Wehrsubstanz verwendet. Kommt auch in Brennnesseln und Tannennadeln vor. |
| Benzoesäure, ihre Salze und ihr Ethylester | Im Benzoeharz (*Styrax benzoin*) und im Wehrsekret von Wasserkäfern (*Dytiscus sp*). |
| Benzylalkohol | Bis zu 6 % im Jasminblütenöl und sowohl frei als auch verestert in vielen anderen ätherischen Ölen. |
| Dehydracetsäure und ihre Salze | In den Blüten des Goldkelchs (*Solandra nitida*, *Solandra grandiflora*). |
| Propionsäure und ihre Salze | Wird bei der Propionsäuregärung gebildet. Kohlenhydrate werden durch *Lactobacillus casei, Bacillus subtilis* oder |
| | *Propionbakterium pentosaceum* zu Propionsäure umgesetzt. |
| Salicylsäure und ihre Salze | Als freie Säure in der Spierstaude (*Filipendula ulmaria*), in Sennesblättern (*Cassia angustifolia*, *Cassia senna*) und in Kamillenblüten (*Chamomilla recutita*)*.* |
| Sorbinsäure und ihre Salze | In den Samen der Vogelbeere (Eberesche, *Sorbus aucuparia*). |

Eine gesetzliche Definition des Begriffs "Naturkosmetik" gibt es weder national noch europaweit. Es gibt jedoch formelle Kriterien, welche Anforderungen an derartige Produkte zu stellen sind. Naturkosmetika sind demnach ausschließlich aus Naturstoffen herzustellen. Naturstoffe sind Substanzen pflanzlichen, tierischen und/oder mineralischen Ursprungs sowie deren Gemische und Reaktionsprodukte.

Für naturkosmetik-konforme Zubereitungen gelten zwar unterschiedliche Kriterien, beispielsweise ist jedoch auch in diesen Kriterien die Zulassung von Konservierungsmitteln geregelt

Die Herausforderung war es einen blauen Farbstoff natürlicher Herkunft zu finden, der in Zwei-Phasenrodukten einsetzbar ist und die Einfärbeproblematik löst.

Die Erfindung ist eine mehr-, insbesondere zweiphasige, kosmetische oder dermatologische Zubereitung umfassend eine Öl- und eine Wasserphase und Guaiazulen

Guaiazulen, 1,4-dimethyl-7-isopropylazulene (CAS 489-84-9), der Struktur 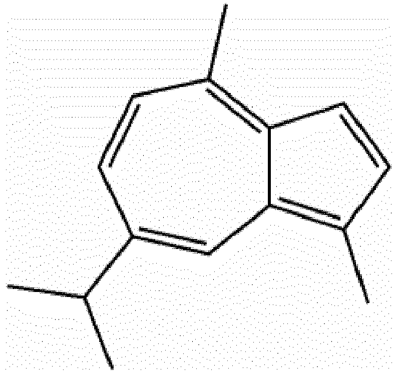 ist ein FDA geprüfter Zusatzstoffstoff in Kosmetika.

Aufgrund seiner blauen Farbe wird er zur Einfärbung kosmetischer Zubereitungen genutzt.

Guaiazulen wird als löslich in Methanol und Paraffinöl beschrieben.

In der DE 10 2005 036 160 A1 wird der Zusatz an Guajazulen als antiphlogistischer oder antibakterieller Wirkstoff zu dermatologischen Zwecken in Emulsionszubereitungen beschrieben.

In der DE 10 2008 040 977 A1 wird Guajazulen als entzündungshemmender Zusatz in Reinigungsmilchprodukten beschrieben.

Überraschend färbt Guaiazulen nur die Lipidphase eines Mehrphasenproduktes, insbesondere des Zweiphasenproduktes, ein und löst damit die gestellte Aufgabe nur eine Phase des Zweiphasenproduktes zu färben.

Darüber hinaus ist Guaiazulen ein Naturstoff und entsprechend obiger Beschreibung als Rohstoff in naturkosmetik-konformen Zubereitungen einsetzbar.

Die Gewinnung und/oder Herstellung des Guaiazulen entspricht den Anforderungen für naturkosmetik-konforme Stoffe. Zudem ist Guaiazulen, gelöst in natürlichen und naturnahen Lipiden, ausreichend farbstabil für den Einsatz in kosmetischen Produkten mit einer typischen Haltbarkeit von 30 Monaten.

Beispielhaft wurde die Stabilität des Guaiazulens in folgender Lipidmischung mittels Suntester untersucht:

| Inhaltsstoff | Konz. [Gew. %] |
|---|---|
| Guaiazulen | 0,016 |
| Dicaprylylether | 4 |
| Rapsöl | 93,934 |
| Jojobaöl | 1 |
| Mandelöl | 1 |
| Tocopherol | 0,05 |

Die Prüfmuster wurden in 20 mL Klarglas-Weithalsgläschen im Suntester bestrahlt.

### Bestrahlungsbedingungen:

Gerät: Atlas Suntester ("Hinterglas"), 121283
Filterkombination: Beschichtetes Quarzglas 56052388, FG 56052372 und ID65 56077769
Bestrahlungsstärke: 250 W/m2
Bestrahlungszeit: 26* h

Die im Suntester 26 Stunden bestrahlte Probe zeigt eine nur geringfügige Farbschwächung gegenüber dem im Dunkeln gelagertes Rückstellmuster und der lichtdicht im Suntester gelagerten Temperaturkontrollprobe.

Die Bestrahlung entspricht näherungsweise der Strahlendosis, mit der ein Produkt am Fenster ohne direkte Sonneneinstrahlung über 6 Monate beansprucht werden würde.

Andere blaue Farbstoffe, wie beispielsweise Indigo, haben den Nachteil, dass sie nicht nur die Ölphase einfärben sondern aufgrund ihrer teilweisen Wasserlöslichkeit auch die Wasserphase einfärben. Damit ist eine klare optische und für den Verbraucher akzeptable Phasentrennung nicht möglich.

Die Anteile an Guaiazulen werden bevorzugt in Bereichen von 0,001 bis 0,02 Gew.%, insbesondere bis 0,008 Gew.%, insbesondere im Bereich von 0,003 bis 0,005 Gew.%, bezogen auf die Gesamtmasse der Zubereitung, gewählt.

Die Zubereitung ist darüber hinaus insbesondere frei von Parabenen, insbesondere 4-Hydroxybenzoesäure und deren Ester, Methylparaben, Ethylparaben, Propylparaben, Isopropylparaben, Butylparaben, Isobutylparaben, Phenylparaben, des Weiteren frei von Benzylalkohol, Formaldehyd, Phenoxyethanol, Ethylenglycolmonophenylether, Phenylglycol, Phenoxetol^{®}, Benzethoniumchlorid, Lauroylethylarginat, Octopirox und/oder Methylisothiazolinon.

Der Anteil an diesen Stoffen beträgt daher weniger als 0,1 Gew.%, insbesondere weniger als 0,01 Gew.%, insbesondere 0 Gew.%, bezogen auf die Gesamtmasse der Zubereitung, um als "frei von" bezeichnet zu werden.

Ethanol ist ebenfalls ein Stoff der bevorzugt in der Zubereitung nicht enthalten ist. Da jedoch oft durch Extrakte o.ä. Rohstoffe geringe Mengen an Ethanol in die Formel eingebracht werden können, ist erfindungsgemäß eine Ethanolfreiheit mit einem Anteil von weniger als 0,1 Gew.%, bezogen auf die Gesamtmasse der Zubereitung, erreicht.

Vorteilhaft kann auf Zusatz von Konservierungsmitteln und Konservierungsmittelhelfer und insbesondere Alkohol gänzlich verzichtet werden.

Die Zubereitung weist einen pH-Wert im Bereich 4,5 bis 7,5, bevorzugt 4,5 bis 5,5 auf.

Der pH-Wert der Zubereitung kann mit Natronlauge eingestellt werden.

In einem Hühnerei-Test an der Chorionallantoismembran (CAM) konnte gezeigt werden, dass mit Hilfe des Guaiazulens eine sehr gute Hautverträglichkeit in naturkosmetik-konformen Zubereitungen erreicht werden konnte.

### HET-CAM:

Dieser Test dient zur vergleichenden Abschätzung der Reizpotentiale von Rohstoffen und Produkten an der Chorio-Allantois-Membran (CAM) von bebrüteten Hühnereiern.

LSL-Eier (Lohmann's Selected Leghorn, Fa. Lohmann) werden am 9. Bruttag am breiten Pol geöffnet, die Eihaut entfernt und 300 µl flüssiges bzw. 100 mg festes Prüfmuster auf die CAM appliziert. Verdünnungen der Prüfmuster werden mit Kochsalzlösung oder Olivenöl hergestellt. Pro Konzentration werden 3 bzw. 4 Eier eingesetzt. Die Untersuchungen werden, abhängig vom Prüfmuster, nach dem Reaktionszeit- oder dem Feststoff-Protokoll durchgeführt. Für Prüfmuster und Verdünnungen, die transparent sind und eine kontinuierliche Beobachtung der CAM gestatten, findet das Reaktionszeit-Protokoll Anwendung (üblicherweise mit unverdünnten und 10%igen Prüfmustern). Hier werden innerhalb von fünf Minuten nach Applikation die Eintrittszeitpunkte der drei Reaktionstypen Blutung, Lysis und Koagulation, sowie ihre Schweregrade durch Beobachtung mit dem bloßen Auge bestimmt. Falls notwendig werden die Reaktionen durch Betrachtung mit einer Stereolupe (10-40 fache Vergrößerung) verifiziert. Mit einer empirisch ermittelten Formel wird aus den Reaktionszeiten der Reizindex berechnet, mit dem eine Klassifizierung des Prüfmusters zwischen "nicht reizend" bis "stark reizend" möglich ist. Zusätzlich wird die Reizschwelle bestimmt, d.h. die Konzentration, bei der die ersten schwachen Reaktionen beobachtet werden. Nichttransparente Verdünnungen bzw. Zubereitungen oder Rohstoffe werden nach dem Feststoff-Protokoll mit 5 Minuten Inkubationszeit untersucht. Nach Entfernung des Prüfmusters von der Applikationsfläche werden die Schweregrade der genannten Reaktionstypen beurteilt und das Prüfmuster entsprechend klassifiziert. Frühe Reaktionszeiten und starke Schweregrade korrelieren mit starken Reizpotentialen. Beide Protokolle sind in einer vom BGA durchgeführten Ringstudie angewendet worden (1988-1991). Abgewandelte Methoden des Hühnerei-Tests wurden in einer von der CTFA initiierten Studie bzw. in einer EC/HO-Studie geprüft.

Nachfolgende Untersuchungen belegen das niedrige Reizpotential erfindungsgemäßer Zubereitungen.

Im HET-CAM Test wurden folgende Zubereitungen untersucht:

| **Inhaltsstoffe** | **Rezeptur 1** | **Rezeptur 2** | **Rezeptur 3** |
|---|---|---|---|
| **Ölphase** | **Gew. %** | **Gew. %** | **Gew. %** |
| Nachtkerzenöl | 0,01 | 0,01 | 0 |
| Dicaprylyether | 2 | 2 | 35 |
| Rapsöl | 31,5826 | 46,825 | 3 |
| Jojobaöl | 0,5 | 0,5 | 0 |
| Mandelöl | 0,5 | 0,5 | 0 |
| Tocopherol | 0,025 | 0,025 | 0,025 |
| Guaiazulen | 0,005 | 0,008 | 0,005 |

| **Wasserphase** | **Gew. %** | **Gew. %** | **Gew. %** |
|---|---|---|---|
| Natronlauge 45% | 0,1 | 0,11 | 0 |
| Dehydracetsäure | 0,2 | 0,2 | 0,2 |
| Lävulinsäure | 0,1 | 0,1 | 0,1 |
| Natriumlävulinat | 0,1 | 0,1 | 0,1 |
| Kochsalz | 1 | 1 | 1 |
| Demineralisiertes Wasser | add. 100% | add. 100% | add. 100% |
| Glycerin | 6,2 | 6,2 | 10,2 |
| Kokosglykosid (Tensid) | 0,5 | 0,5 | 0 |

Es wurden sowohl die Zubereitungen geschüttelt als temporäre Emulsion als auch die Ölphase mit Guaiazulen und Wasserphase getrennt untersucht.

Die Ölphasen mit Guaiazulen alleine erzeugten jeweils keine Reaktionen. Lediglich die Mischungen und Wasserphasen verursachten aufgrund des Glycerin- bzw. Tensidgehaltes Blutstauungen bzw. schwache bis mäßige intravasale Koagulationen. Daher kann nur bei dem Einsatz von höheren Mengen Glycerin (größer 10%) und Tensiden ein Reizpotential für die Augen nicht ausgeschlossen werden. Bevorzugt ist der Glyceringehalt daher unter 10 Gew.%, insbesondere unterhalb von 6 Gew.%, bezogen auf die Gesamtmasse der Zubereitung, zu wählen.

| Reaktionen im HET-CAM-Test | Rezeptur 1 | Rezeptur 2 | Rezeptur 3 |
|---|---|---|---|
| Ölphase allein | keine | keine | keine |
| Wasserphase | 2x mäßige, 1x schwache Blutungen*, 1x mäßige, 1x schwache intravasale Koagulationen, 2x keine | 3x mäßige, 1x schwache Blutungen*, 1x mäßige, 1x schwache intravasale Koagulationen, 2x keine | 3x mäßige, 1x schwache Blutungen*, 1x mäßige, 1x schwache intravasale Koagulationen, 2x keine |
| Geschüttelt | 2x mäßige, 1x schwache Blutungen*, 1x mäßige, 1x schwache intravasale Koagulationen, 2x keine | 3x mäßige, 1x schwache Blutungen*, 1x mäßige, 1x schwache intravasale Koagulationen, 2x keine | 3x mäßige, 1x schwache Blutungen*, 2x mäßige intravasale Koagulationen, 2x keine |

| | | | |
|---|---|---|---|
| *Es handelte sich bei mikroskopischer Kontrolle um Blutstauungen, die makroskopisch als Blutungen erscheinen. | | | |

Bei dem Verzicht auf Glycerinkonzentrationen über 10% und dem Einsatz von Tensiden kann mit dieser Art von Zubereitungen eine sehr gute Haut- und Augenverträglichkeit erreicht werden. Dieses wurde auch anhand einer dermatologischen und ophthalmologischen Studie verifiziert. Die folgende Zubereitung haben dazu 33 Probanden über 2 Wochen täglich angewendet:

| Inhaltsstoffe | Gew.% |
|---|---|
| Guaiazulen | 0,005 |
| Dicaprylylether | 13 |
| Rapssamenöl | 16 |
| Glycerin | 5 |
| Natriumhydroxid | 0,04 |
| Dehydracetsäure | 0,05 |
| Lävulinsäure | 0,1 |
| Natriumlävulinat | 0,1 |
| Kochsalz | 1 |
| Lotusextrakt | 0,1 |
| Wasser demineralisiert | add. 100% |

Der Haut- und Augenzustand wurde vor der ersten Anwendung und nach 2 Wochen von einem Dermatologen und einem Ophthalmologen bewertet.

Die erfindungsgemäßen Zubereitungen sind vorteilhaft frei von anderen Konservierungsmitteln, insbesondere Benzylalkohol-, Paraben- und Phenoxyethanolfrei und weisen dennoch eine ausreichende mikrobielle Stabilität auf.

Zur Konservierung kann Dehydracetsäure und Lävulinsäure in den erfindungsgemäßen Formulierungen enthalten sein.

Die kosmetischen Zubereitungen gemäß der Erfindung können ferner kosmetische Hilfsstoffe und weitere Wirkstoffe enthalten, wie sie üblicherweise in solchen Zubereitungen verwendet werden, z. B. Substanzen zum Verhindern des Schäumens, weitere Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate sofern der Zusatz die geforderten Eigenschaften hinsichtlich der Einfärbung und ggf. mikrobiologischen Stabilität sowie Haut- und Haarverträglichkeit nicht beeinträchtigt bzw. deren Anteil zu vermeiden ist.

Die erfindungsgemäßen Zubereitungen umfassen vorzugsweise nur Naturstoffe, natürliche, naturnahe und/oder naturidentische Rohstoffe. Naturstoffe sind Substanzen pflanzlichen, tierischen und/oder mineralischen Ursprungs sowie deren Gemische und Reaktionsprodukte. Als natürliche, naturnahe und/oder naturidentische Rohstoffe sind Stoffe anzusehen, die aus Naturstoffen nur durch physikalische Mittel gewonnen werden, nur chemisch verändert werden bzw. naturidentisch hergestellt werden.

Entscheidender Unterschied zwischen den erfindungsgemäßen Zubereitungen und Emulsionszubereitungen ist die Phasentrennung.

Unter einer Emulsion versteht man ein fein verteiltes Gemisch zweier normalerweise nicht mischbarer Flüssigkeiten ohne sichtbare Entmischung.

Ein mehr- bzw. zweiphasige Zubereitung hat aber gerade dieses Merkmal der sichtbaren Entmischung, ansonsten es keine Mehrphasenzubereitung sein kann.

Für den Fachmann der Kosmetik ist damit klar zu unterscheiden zwischen einer Emulsion und einer Zweiphasenzubereitung.

Eine Zweiphasenzubereitung wird allein durch Energieeintrag, wie Schütteln, zu einer Mischung. Nach kürzester Zeit entmischt sich aber eine Zweiphasenzubereitung wieder. Eine kosmetische Emulsion ist jedoch langzeitstabil ohne Entmischung.

Guaiazulen kann vorteilhaft daher zur Einfärbung der Lipidphase von mehrphasigen, insbesondere zweiphasigen, kosmetischen oder dermatologischen Zubereitungen, umfassend mindestens eine Lipid- und Wasserphase, verwendet werden.

Nachfolgendes Beispiel veranschaulicht die erfindungsgemäßen Zubereitungen. Die Angaben sind Gewichtsanteile jeweils bezogen auf die Gesamtmasse der Zubereitung.

### Beispiel

| **Inhaltsstoffe** | **Gew.%** |
|---|---|
| Guaiazulen | 0,005 |
| Dicaprylylether | 13 |
| Rapssamenöl | 16 |
| Glycerin | 5 |
| Natriumhydroxid | 0,04 |
| Dehydracetsäure | 0,05 |
| Lävulinsäure | 0,1 |
| Natriumlävulinat | 0,1 |
| Kochsalz | 1 |
| Wasser demineralisiert | add. 100% |

## Patentansprüche

1. Mehrphasige kosmetische oder dermatologische Zubereitung umfassend mindestens eine Öl- und mindestens eine Wasserphase und Guaiazulen.

2. Zweiphasige Zubereitung kosmetische oder dermatologische Zubereitung umfassend eine Öl- und eine Wasserphase und Guaiazulen.

3. Zubereitung nach Anspruch 1 oder 2 **dadurch gekennzeichnet, dass** der Anteil an Guaiazulen im Bereich von 0,001 bis 0,02 Gew.%, insbesondere bis 0,008 Gew.%, insbesondere im Bereich von 0,003 bis 0,005 Gew.%, bezogen auf die Gesamtmasse der Zubereitung, gewählt wird.

4. Zubereitung nach einem der vorstehenden Ansprüche **dadurch gekennzeichnet, dass** die Zubereitung frei ist von Parabenen, Benzylalkohol, Formaldehyd, Phenoxyethanol, Ethylenglycolmonophenylether, Phenylglycol, Benzethoniumchlorid, Lauroylethylarginat, Octopirox und/oder Methylisothiazolinon.

5. Zubereitung nach einem der vorstehenden Ansprüche umfassend Dehydracetsäure und/oder Lävulinsäure.

6. Zubereitung nach einem der vorstehenden Ansprüche 1 bis 5 umfassend neben Guaiazulen nur Naturstoffe, natürliche, naturnahe und/oder naturidentische Rohstoffe.

7. Zubereitung nach einem der vorstehenden Ansprüche **dadurch gekennzeichnet, dass** der pH-Wert der Zubereitung im Bereich 4,5 bis 7,5, bevorzugt 4,5 bis 5,5 ausgewählt wird.

8. Zubereitung nach einem der vorstehenden Ansprüche umfassend weniger als 10 Gew.% Glycerin, bevorzugt weniger als 6 Gew.%, bezogen auf die Gesamtmasse der Zubereitung.

9. Zubereitung nach einem der vorstehenden Ansprüche umfassend weniger als 0,1 Gew.% Ethanol, bezogen auf die Gesamtmasse der Zubereitung.

10. Verwendung von Guaiazulen zur Einfärbung der Lipidphase von mehrphasigen kosmetischen oder dermatologischen Zubereitungen umfassend mindestens eine Lipid- und Wasserphase.
